# EUROPEAN PATENT SPECIFICATION

(11) **EP 0 373 683 B1**
(45) Date of publication and mention of the grant of the patent: **23.02.1994**
(21) Application number: 89202814.3
(22) Date of filing: 07.11.1989
(51) Int. Cl.: C07D 301/32, B01D 53/22, B01D 69/12

(54) **A process for the separation of carbon dioxide**
Verfahren zur Abtrennung von Kohlendioxid
Procédé de séparation du dioxyde de carbone

(30) Priority: 22.11.1988 GB 8827265
(43) Date of publication of application: 20.06.1990
(73) Proprietor: SHELL INTERNATIONALE RESEARCH MAATSCHAPPIJ B.V., 2596 HR Den Haag (NL)
(72) Inventor: Schuurmans, Hubertus Johanna Adrianus, NL-1031 CM Amsterdam (NL); Bitter, Johan George Albert, NL-1031 CM Amsterdam (NL)

(56) References cited:
- EP-A- 0 134 055
- EP-A- 0 200 518
- EP-A- 0 254 556

## Description

The invention relates to a process for the separation of carbon dioxide gas from a mixture comprising components resulting from the reaction of ethylene and oxygen under the influence of a silver catalyst.

It is generally known that in the preparation of ethylene oxide by the reaction of ethylene and oxygen with a silver catalyst by-products are formed. A major by-product formed is carbon dioxide, which is largely responsible for the fact that the selectivity to ethylene oxide only reaches about 80 to 82 mol%.

It is undesirable that large amounts of carbon dioxide are present in the unreacted components which are recycled to the oxidation. Usually, after the ethylene oxide product has been removed from the reaction mixture by absorption in water, the remaining mixture of gases containing unreacted compounds and diluents, which may contain, for example, methane, nitrogen, argon, oxygen, ethylene, ethane and carbon dioxide, is partially freed from carbon dioxide. For this purpose the reaction mixture is contacted with absorbants, such as alkali metal carbonates, especially potassium carbonate, in a scrubber. It is believed that the reaction that occurs during absorption is K₂CO₃ + H₂O + CO₂ ---> 2KHCO₃.

Since regeneration of potassium carbonate is necessary for environmental reasons and from the viewpoint of cost, a large amount of steam is used for regeneration, thus adding to the overall cost of the process.

In addition, it is also necessary to remove argon which builds up in the recycle stream. The conventionally employed bleeding of the argon results in a concurrent loss of ethylene with a consequent disadvantageous effect on the overall process economics.

The applicant has now found that the carbon dioxide can be removed from the reaction mixture in a cheaper and cleaner way, obviating the need for absorption with a carbonate and subsequent desorption. Furthermore it has been found that the need for an argon bleed can be minimized, thus reducing the potential loss of ethylene.

The invention relates to a process for the separation of carbon dioxide gas from a mixture comprising components resulting from the reaction of ethylene and oxygen under the influence of a silver catalyst wherein the separation is carried out by absorption of the carbon dioxide gas into one wall of a membrane, solubilization in the membrane matrix, diffusion through the membrane and desorption from the other wall.

Membranes are very well described in Kirk-Othmer "Encyclopaedia of Chemical Technology", third edition part 15, under the heading "MEMBRANE TECHNOLOGY", especially pages 102 and 104 describing dense membranes and porous membranes respectively.

A very important and fundamental means by which a species can be transported through a membrane involves dissolving of the permeate molecules into the membrane at its upstream surface, followed by molecular diffusion down its concentration gradient to the downstream face of the membrane. There it is evaporated or dissolved into the adjacent fluid phase. The driving force for diffusion through the membrane is the pressure exerted on the system. Another driving force is the concentration. In fact it is the pressure differential and the concentration differential between the upstream and downstream surfaces of the membrane which constitute the major driving forces.

Preferred membranes to be used in the process for the separation of carbon dioxide in accordance with the present invention are the dense membranes. Dense membranes generally have the ability to transport species selectively and are therefore applicable for molecular separation processes, such as gas purification. With dense membranes, even molecules of the same size can be separated when their solubilities or diffusivities in the membrane differ. Dense membranes may have low transport rates. To attain acceptable transport rates, required for commercial application in separation processes where productivity is of paramount importance, it is necessary to make the membranes ultrathin.

The pressure exerted on the upstream face of the membrane is generally in the range of from 1 to 100 bar (100 to 10000 kPa) during separation. Preferred pressures lie within the range of from 10 to 80 bar (1000 to 8000 kPa).

Preferably the mixture from which carbon dioxide is separated, comprises those components resulting from the reaction of ethylene and oxygen under the influence of a silver catalyst after removal of product ethylene oxide, preferably by washing with water. In practice the main components of the mixture are selected from carbon dioxide, methane, ethylene, argon, nitrogen and oxygen, dependent on the starting mixture used for the oxidation process.

The gaseous mixture remaining after separation of carbon dioxide in accordance with the invention can be recycled to the oxidation. It has been found that at least a proportion of any argon present is also separated with the carbon dioxide, thus reducing the need for a bleed to prevent argon build up.

Preferably, the dense membrane used in the process according to the invention is a membrane obtained by plasma polymerization. Plasma, in physics, is considered to be a collection of positively and negatively charged particles and neutral species (molecules, atoms and radicals), forming a neutrally charged distribution of matter. Plasmas can exist in solids (as excited electrons in metals) and liquids (as salt dissolved in water) but are usually considered more closely related to gases.

When energy (e.g. heat) is continuously added to a solid, it first melts, then vaporizes, and finally electrons are removed from some of the neutral gas atoms and molecules (a process called ionization) to produce a mixture of positive ions and (negative) electrons, while overall neutral charge density is maintained. When a significant portion of the gas has been ionized, its characteristics will be substantially altered and will bear little resemblance to solids, liquids and gases. Plasma state can be considered as the fourth state of matter and is unique in the way in which it interacts with itself, with electric and magnetic fields, and with its environment.

Plasma polymerization is a process wherein organic monomers are introduced into a space filled with a plasma, whereby the organic monomers are activated, for example by applying an electric field, and are converted into radicals or ions to effect polymerization. Membranes comprising one or more layers of plasma polymerizate can be made. The plasma polymerizate is usually applied on a porous substrate.

The film of plasma polymerizate may be formed from any monomeric organic compound of an ionizable nature. Suitable examples of such organic compounds are olefins, aromatics, alkylene oxides, halogenated lower hydrocarbons and nitriles. Preferably, such an organic compound together with an inert gas, e.g. argon, is brought into a plasma chamber surrounded by an inductive coil or provided with electrodes. Various modes of reaction take place simultaneously in plasma polymerization.

In the present case, preferably a dense, highly permeable intermediate layer is present in between the film of plasma polymerizate and the porous substrate. This intermediate layer serves two purposes, viz. support of the plasma polymerizate and distribution of fluid over the porous substrate. The mechanical stability of the intermediate layer enables the application of a very thin top layer formed by the plasma polymerizate. The second function of the intermediate layer, i.e. distribution of the fluid passed through the dense selective film of plasma polymerizate, allows the whole area of the said latter film to be effectively used for fluid separation, despite the presence of the porous substrate.

The membrane discussed hereinbefore thus has three layers, viz. a dense, ultrathin, selective film of plasma polymerizate, a dense highly permeable intermediate layer and a microporous substrate supporting both layers.

Such three layer membranes are fully described in European Patent Application 134055.

It has been found that such membranes have an excellent permeability for carbon dioxide gas and a low permeability for ethylene. Consequently a gaseous mixture comprising carbon dioxide and ethylene can be transformed into a mixture comprising only a fraction of the original carbon dioxide and almost all ethylene originally present. If desired, in the interests of minimizing ethylene loss, the permeate gas containing a high percentage of carbon dioxide and a low percentage of ethylene may be subjected again to a second membrane process. The second permeate can be disposed of by suitable means.

The invention is illustrated by the following examples.

### Example 1

A layer of plasma polymerizate was prepared by loading a gas mixture through an electric discharge chamber (forming cold plasma) over a composite membrane substrate consisting of a microporous polypropylene layer covered on the plasma-facing side with a dense, permeable layer of polydimethylsiloxane. The gas mixture comprised toluene and argon in a volume ratio of 1:4. The following conditions were applied:

| | |
|---|---|
| temperature | 20°C |
| argon flow | 0.38 cm³ per min |
| chamber pressure | 5 Pa |
| power | 4 W |
| duration | 5 min |

A three layer composite membrane was obtained, of which the plasma layer had a thickness of 16.7 nanometer.

Said membrane was tested at a gas feed pressure of 1400 kPa on one side of the membrane and atmospheric pressure on the downstream side of the membrane (surface area 100 cm²) at a temperature of 25°C. The total flux was 1.2 normal m³/m².bar.d.

The feed consisted of 6.3 %mol of ethylene, 48.3 %mol of carbon dioxide and 45.3 %mol of methane. The permeate gas mixture consisted of 0.7 %mol of ethylene, 96.2 %mol of carbon dioxide and 3.1 %mol of methane. No deterioration in performance was observed during the test which lasted one week.

### Example 2

A three layer composite membrane was prepared somewhat similar to that described in Example 1 but having a plasma layer of thickness of 45.5 nanometer.

The membrane was tested at a gas feed pressure of 1700 kPa on one side of the membrane and a pressure of 100 kPa (1 bar) at the permeate side of the membrane (surface area 100 cm²) at a temperature of 21°C. The total flux was 3.0 normal m³/m².bar.d.

The feed, which was the effluent from a process for producing ethylene oxide by direct oxidation of ethylene with oxygen after removal of product ethylene oxide, consisted of a mixture of 24.3 %mol of ethylene, 6.4 %mol of carbon dioxide, 56.5 %mol of methane, 8.2 %mol of argon and 4.6 %mol of oxygen. The permeate gas mixture consisted of 11.2 %mol of ethylene, 46.3 %mol of carbon dioxide, 22.0 %mol of methane, 10.4 %mol of argon and 10.1 %mol of oxygen. No deterioration in performance was observed during the test which lasted four weeks.

This example illustrates the transfer of argon, together with carbon dioxide, to the permeate gas mixture.

## Claims

1. A process for the separation of carbon dioxide gas from a mixture comprising components resulting from the reaction of ethylene and oxygen under the influence of a silver catalyst wherein the separation is carried out by absorption of the carbon dioxide gas into one wall of a membrane, solubilization in the membrane matrix, diffusion through the membrane and desorption from the other wall.

2. A process according to claim 1, wherein a dense membrane is used.

3. A process according to claim 1 or 2, wherein the membrane is obtained by plasma polymerization.

4. A process according to claim 3, wherein a membrane is used comprising a dense selective film of a plasma polymerizate, a dense highly permeable intermediate layer and a microporous substrate supporting the plasma polymerizate film and the intermediate layer.

5. A process according to any one of the claims 1-4, wherein the separation is carried out at a pressure in the range of from 1 to 100 bar (100 to 10000 kPa).

6. A process according to any one of the claims 1-5, wherein the mixture comprises components resulting from the reaction of ethylene and oxygen under the influence of a silver catalyst after the main product ethylene oxide has been removed.

7. A process according to claim 6, wherein the ethylene oxide has been washed out with water.

8. A process according to any one of the preceding claims, wherein, in addition to carbon dioxide, the components are selected from methane, ethylene, argon, nitrogen and oxygen.

9. A process according to claim 8 wherein the components comprise argon which is at least partially separated with the carbon dioxide.

10. A process for the preparation of ethylene oxide by the reaction of ethylene and oxygen under the influence of a silver catalyst, wherein carbon dioxide is separated from components resulting from the reaction by the process of any one of the preceding claims.

11. A process according to claim 10 wherein the gaseous mixture from which carbon dioxide has been separated is recycled to the ethylene oxidation.

## Patentansprüche

1. Ein Verfahren zur Abtrennung von Kohlendioxidgas aus einem Gemisch, umfassend Komponenten, die aus der Reaktion von Ethylen und Sauerstoff unter Einfluß eines Silberkatalysators stammen, in dem die Abtrennung durch Absorption des Kohlendioxidgases in eine Wand einer Membran, Solubilisierung in der Membranmatrix, Diffusion durch die Membran hindurch und Desorption aus der anderen Wand durchgeführt wird.

2. Ein Verfahren gemäß Anspruch 1, in dem eine dichte Membran verwendet wird.

3. Ein Verfahren gemäß Anspruch 1 oder 2, in dem die Membran durch Plasmapolymerisierung erhalten wird.

4. Ein Verfahren gemäß Anspruch 3, in dem eine Membran verwendet wird, umfassend einen dichten selektiven Plasmapolymerisatfilm, eine dichte, hoch durchlässige Zwischenschicht und ein mikroporöses Substrat , welches den Plasmapolymerisatfilm und die Zwischenschicht stützt.

5. Ein Verfahren gemäß irgendeinem der Ansprüche 1 bis 4, in dem die Abtrennung bei einem Druck im Bereich von 1 bis 100 bar (100 bis 10000 kPa) durchgeführt wird.

6. Ein Verfahren gemäß irgendeinem der Ansprüche 1 bis 5, in dem das Gemisch Komponenten umfaßt, die aus der Reaktion von Ethylen und Sauerstoff unter Einfluß eines Silberkatalysators nach Abziehen des Haupterzeugnisses Ethylenoxid erhalten wurden.

7. Ein Verfahren gemäß Anspruch 6, in dem Ethylenoxid mit Wasser ausgewaschen worden ist.

8. Ein Verfahren gemäß irgendeinem der vorhergehenden Ansprüche, in dem, zusätzlich zum Kohlendioxid, die Komponenten aus Methan, Ethylen, Argon, Stickstoff und Sauerstoff gewählt werden.

9. Ein Verfahren gemäß Anspruch 8, in dem die Komponenten Argon umfassen, welches zumindest teilweise zusammen mit dem Kohlendioxid abgetrennt wird.

10. Ein Verfahren zur Herstellung von Ethylenoxid durch die Reaktion von Ethylen und Sauerstoff unter Einfluß eines Silberkatalysators, in dem Kohlendioxid von Komponenten, die aus der Reaktion stammen, durch das Verfahren gemäß irgendeinem der vorhergehenden Ansprüche abgetrennt wird.

11. Ein Verfahren gemäß Anspruch 10, in dem das gasförmige Gemisch, aus dem das Kohlendioxid abgetrennt worden ist, zur Ethylenoxidation zurückgeführt wird.

## Revendications

1. Procédé pour la séparation du dioxyde de carbone gazeux à partir d'un mélange comprenant des composants résultant de la réaction de l'éthylène et de l'oxygène sous l'influence d'un catalyseur à base d'argent dans lequel on effectue la séparation par absorption du dioxyde de carbone gazeux dans une paroi d'une membrane, la solubilisation dans la matrice de la membrane, la diffusion à travers la membrane et la désorption à partir de l'autre paroi.

2. Procédé selon la revendication 1, dans lequel on utilise une membrane dense.

3. Procédé selon la revendication 1 ou 2, dans lequel on obtient la membrane par polymérisation en plasma.

4. Procédé selon la revendication 3, dans lequel on utilise une membrane comprenant un film dense sélectif d'un polymérisat en plasma, une couche intermédiaire dense hautement perméable et un substrat microporeux supportant le film de polymérisat en plasma et la couche intermédiaire.

5. Procédé selon l'une quelconque des revendications 1 à 4, dans lequel on effectue la séparation à une pression dans la gamme de 1 à 100 bars (1000 à 10 000 kPa).

6. Procédé selon l'une quelconque des revendications 1 à 5, dans lequel le mélange comprend des composants résultant de la réaction de l'éthylène et de l'oxygène sous l'influence d'un catalyseur à base d'argent après que l'on a supprimé l'oxyde d'éthylène principal produit.

7. Procédé selon la revendication 6, dans lequel on a lavé l'oxyde d'éthylène avec de l'eau.

8. Procédé selon l'une quelconque des revendications précédentes, dans lequel, en plus du dioxyde de carbone, on choisit les composants parmi le méthane, l'éthylène, l'argon, l'azote et l'oxygène.

9. Procédé selon la revendication 8, dans lequel les composants comprennent de l'argon qui est au moins partiellement séparé avec le dioxyde de carbone.

10. Procédé pour la préparation d'oxyde d'éthylène par la réaction de l'éthylène et de l'oxygène sous l'influence d'un catalyseur à base d'argent, dans lequel on sépare le dioxyde de carbone des composants résultants de la réaction à l'aide d'un procédé selon l'une quelconque des revendications précédentes.

11. Procédé selon la revendication 10 dans lequel le mélange gazeux à partir duquel le dioxyde de carbone a été séparé est recyclé vers l'oxydation de l'éthylène.
